# EUROPEAN PATENT SPECIFICATION

(11) **EP 1 834 592 B1**
(45) Date of publication and mention of the grant of the patent: **14.09.2016**
(21) Application number: 07251073.8
(22) Date of filing: 14.03.2007
(51) Int. Cl.: A61B 17/04

(54) **Apparatus for arthroscopic surgery using suture anchors**
Vorrichtung für arthroskopische Chirurgie unter Verwendung von Nahtankern
Appareil pour chirurgie arthroscopique utilisant des ancrages de suture

(30) Priority: 15.03.2006 US 782556 P; 07.04.2006 US 400704
(43) Date of publication of application: 19.09.2007
(73) Proprietor: DePuy Mitek, LLC, Raynham, MA 02767 (US)
(72) Inventor: Deutsch, Allen, Bellaire, Texas 77401 (US)
(74) Representative: Tunstall, Christopher Stephen

(56) References cited:
- WO-A-00/54666
- WO-A-2004/062507
- WO-A-2006/044491
- US-A- 5 152 790
- US-A- 5 891 168
- US-A1- 2002 052 629
- US-A1- 2002 147 463
- US-A1- 2004 039 404
- US-A1- 2005 283 158
- US-A1- 2006 004 364

## Description

### FIELD OF THE INVENTION

This invention relates to surgical kits for arthroscopic surgery in sports medicine, and more particularly to soft tissue repair using suture anchor devices.

### BACKGROUND OF THE INVENTION

Connective tissue damage or degeneration in the rotator cuff of a patient's shoulder is an often occurring medical condition, in many cases requiring surgical repair including the partial or complete reattachment of torn or detached connective tissue to bone. The terms connective tissue and soft tissue are used interchangeably herein; similarly, the terms suture anchor and suture anchor device are used interchangeably. Conventional suture anchors are devices that are typically implanted in bore holes drilled into bone, and which have conventional surgical sutures affixed in some manner thereto. The suture anchors typically have features which cause the anchors to be engaged in the bone bore hole. Rotator cuff repairs can be performed using open or arthroscopic surgical techniques and procedures, with the minimally invasive arthroscopic methods increasing in acceptance over recent years. Minimally invasive surgical procedures are known to have numerous benefits, including reduced procedure time, reduced infection and scarring, faster patient recovery time, and often improved surgical outcomes. Arthroscopic rotator cuff repair procedures typically employ one or more suture anchors implanted and positioned near the margin of the tear or other damage, to approximate the soft tissue to bone. Arthroscopic rotator cuff repair surgeries are minimally invasive surgical procedures requiring specific surgeon skill sets, and generally requiring the use of suture anchors and other surgical implants or tools that are specially designed for use in arthroscopic procedures.

Although clinical outcomes of arthroscopic rotator cuff repair procedures are typically satisfactory and successful, it is known that there may be subsequent failures and recurrent tears. For example, a suture used to attach torn rotator cuff soft tissue to a suture anchor may cut through the reattached tissue under stress. In addition, the natural footprint, that is, the shape and size of the original biological attachment between the soft tissue and the bone, may not be optimally reproduced to encourage optimal healing in certain cases using present suture anchor-based surgical techniques.

US2004/039404 discloses a suture anchor attached to a tissue-fixation disk with at least one suture knot which resides in a recess in the suture anchor, rather than on the exposed surface of the tissue-fixation disk. The suture that connects the tissue-fixation disk to the suture anchor is looped through the tissue-fixation disk and through at least one suture passage formed through the proximal end of the suture anchor, the free ends of the suture being knotted together within the suture passage of the suture anchor.

US5152790 discloses a ligament graft replacement system having an anchor assembly on one end. The anchor assembly includes an insert member, a threaded sleeve and a rotatable ring to which the graft is attached. A harvested or synthetic graft used as a replacement for a torn ligament or tendon is secured to the rotatable ring by sutures and the anchor assembly is screwed into a tunnel drilled in a bone or bone-like structure. The anchor assembly includes a hexagonal opening through its length for receiving a hex driver in its trailing end or its leading end.

WO00/54666 discloses a free loop non-knot suture assembly for attachment of tissue to bone mass. The assembly includes a continuous suture loop, and an anchor means for capturing portions of the loop with a snag means or recess thereon or therein the anchor means. Once the loop is captured, the anchor is inserted secured into a bone mass which facilitates in a repair of the torn away soft tissue.

US5891168 discloses a knotless suture anchor assembly having a snag element or recess attached to, or formed in, an anchor assembly, for capturing a looped section of the suture element for knotless surgical soft tissue reattachment to bone, and a method for using a plurality of anchors and attachments to provide such attachment or reattachment. A number of suture anchors are "chained" together in a single line. The suture anchor has both a suture loop and "snag means" for capturing the suture loop of an adjacent suture anchor. Thus, the suture anchor has a dual function. The suture anchor has a body that defines a longitudinal axis. The suture anchors can be ribbed, beaded, threaded, or expandable on their exterior surface or can include one or more prongs for secure mating with a bone mass.

A significant need exists in this art for novel methods for arthroscopic rotator cuff repair that overcome some of the deficiencies of the prior art. In addition, there is a need for novel suture anchors and kits to support the surgeon's selection of appropriate suture anchors and associated tools and components for optimal rotator cuff repairs.

### SUMMARY OF THE INVENTION

Accordingly, examplary methods and apparatus for arthroscopic repair of soft tissue damage or degeneration, such as rotator cuff tears are disclosed.

The present invention provides a surgical procedure kit. The kit provides an externally threaded suture anchor having distal and proximal ends, and one or more suture loops affixed near the proximal end. Optionally, there are two suture loops. The kit also provides a first anchor inserter for fixing the first anchor into bone. The kit further provides a second suture anchor having an anchor body with a suture capture notch for receiving a portion of the one or more suture loop, and second suture anchor inserter for fixing the second anchor into bone. One or both of the first and the second suture anchors is bioabsorbable. The second anchor is an interference type suture anchor, or the second anchor has an externally screw-threaded outer sleeve and an inner plug including a distal suture capture notch the second anchor may be a knotless anchor. The kit may optionally have a bone drill and a thread-cutting bone tap, for preparing a hole in the bone to receive one of the first anchor and the second anchor.

The kit may be used in an exemplary method for attaching soft tissue to bone. The examplary method provides for implanting and fixing the first anchor in bone and passing each of the at least one suture loops through the soft tissue. examplary method also provides a second suture anchor having an anchor body that includes a notch for receiving suture. A portion of one of the one or more suture loops is captured in the notch. The second suture anchor is fixed in the bone, wherein fixing the second anchor in the bone is effective to tension the one of the one or more suture loops, for approximating the soft tissue to the surface of the bone. Prior to fixing the at least one of the first suture anchor and the second suture anchor into bone, includes bore holes are formed in the bone to receive the suture anchors.

These and other aspects and features of the present invention will be more apparent from the following description and accompanying drawings.

### BRIEF DESCRIPTION OF THE DRAWINGS

FIG. 1A illustrates a medial row suture anchor installation in a dual row rotator cuff repair.
FIG. 1B illustrates a suture anchor forming part of the kit of the present invention.
FIG. 2a illustrates suture passing in a dual row rotator cuff repair.
FIG. 2B illustrates an alternative suture passing step.
FIG. 3 illustrates a lateral row suture anchor installation in a dual row rotator cuff repair.
FIG. 4A is a top view of one embodiment of a completed dual row rotator cuff repair.
FIG. 4B is a cross-sectional side view of a completed dual row rotator cuff repair.
FIG. 5A illustrates a two-part interference type fixation anchor for dual row rotator cuff repairs.
FIG. 5B illustrates a side-capture two-part interference type fixation anchor for dual row rotator cuff repairs.
FIG. 5C illustrates an externally threaded two-part anchor for dual row rotator cuff repairs.
FIG. 5D illustrates a suture anchor having a suture threader.

### DETAILED DESCRIPTION OF THE INVENTION

The present invention provides surgical kits for performing arthroscopic procedures to repair damaged or degenerated soft tissue, for example, for approximating damaged shoulder rotator cuff tendons (a type of soft tissue) to the humerus. These procedures are referred to as rotator cuff repairs. By the term approximating is meant the bringing two sections of tissue together and into contact. The novel kits of the present invention provide for performing rotator cuff repairs using two rows of one or more suture anchors to approximate soft tissue to bone, typically with a medial row of suture anchors fixed in bone medial to a tear, and a lateral row of anchors fixed in bone relatively lateral to the tear. This type of surgical procedure is referred to herein as a dual row rotator cuff repair, or a dual row repair.

Relative to rotator cuff repairs using a single row of one or more anchor (single row rotator cuff repair, or single row repair), dual row repairs may provide for a larger and better controlled contact area between the reattached tissue and the humerus. It is also to be understood that a dual row repair can be performed in ether a medial-to-lateral direction (medial anchors fixed in bone before medial anchors) or a lateral-to-medial direction (lateral anchors fixed in bone before medial anchors). With that understanding, embodiments are generally described herein in the medial-to lateral direction.

One object of the novel kits of the present invention is to provide improved rotator cuff repairs that better replicale the natural, biological footprint of an undamaged rotator cuff on the humerus than single row repairs. Another object of the present invention is to provide akit for rotator cuff repairs that provide improved initial fixation of the rotator cuff to the surface of the humerus, to reduce the possibility of recurring damage early in the healing process. Yet another object is to provide a kit for providing a distribution of suture anchors and interconnecting sutures over a two-dimensional area of bone and soft tissue in proximity to the tear, thereby reducing the possibility of sutures cutting through (i.e., commonly referred to as "cheesewiring") the approximated soft tissue. Still another object of the present invention is to provide novel surgical procedure kits for useful in performing dual row rotator cuff repair procedures arthroscopically. A further object of the present invention is to provide knotless arthroscopic repair kits for approximating damaged or degenerated soft tissue to bone.

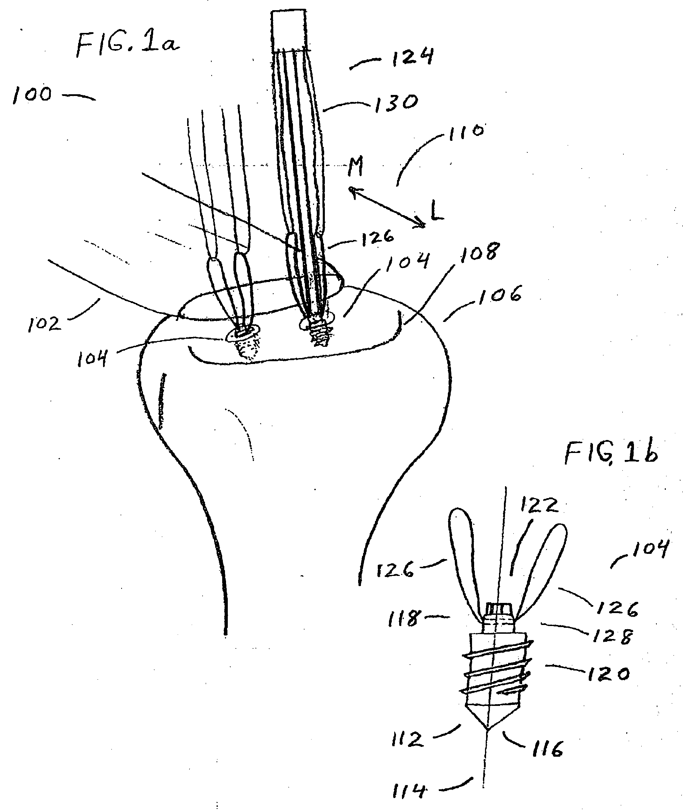

FIGS. 1-4 illustrate an exemplary method for dual row rotator cuff repair. Arthroscopic porlals to the surgical repair site, e.g., including cannulas, are established using conventional surgical techniques, and are not illustrated in the figures herein except where required for descriptions of particular embodiments. Similarly, surgical assessment and preparation of the damaged rotator cuff tendon and the attachment site for rotator cuff repairs are performed using conventional surgical techniques and are not illustrated or discussed herein.

A medial row suture anchor installation 100 in a dual row rotator cuff repair for a detached rotator cuff tendon 102 (soft tissue) is seen in FIG 1A. The method provides for the installation of one or more medial suture anchors 104 in examplary a humerus 106, positioned my dial to the articular margin 108. Medial (M) and lateral (L) directions are illustrated by arrows 110. The detached rotator cuff tendon 102 is shown in a retracted position to provide direct access to medial placement of the medial anchors 104 in the humerus 106. As illustrated in FIG. 1B, the medial anchor 104 is seen to have a body 112 with a longitudinal central axis 114, a distal end 116, a proximal end 118, and external screw threads 120 about the axis 114 extending from the external surface 115 for rotational engagement with bone, for fixation in the humerus 106. the medial suture anchor 104 is typically referred to as a screw threaded type fixation suture anchor.

The medial suture anchor 104 also includes a proximal drive head 122 for mating engagement with an elongated medial anchor insertion tool 124 for fixing (i.e., implanting) the medial suture anchor 104 in bone. In one embodiment, the proximal drive head 124 has a hexagonal cross section about the central axis 114. In another embodiment, the proximal drive head 124 has an oval cross section about the central axis 114. Other types of conventional drive arrangements may also be used including slots, cavities and the like. Optionally, the suture anchors 104 may be provided for use by the surgeon premounted to a respective anchor insertion tool 124 as part of a surgical kit.

The medial suture anchor 104 can be bioabsorbable or nonabsorbable. A bone drill may be used to form a bone bore hole for receiving each medial suture anchor 101. As a further alternative, a thread-cutting bone tap may be is used to prepare internal threads in the bone hole for receiving a medial suture anchor 104. As another alternative, e medial anchor 104 is driven directly into bone without prior drilling of a bone hole.

Affixed proximally to each medial suture anchor is one or more operative suture loops 126 (suture sloop). By the term suture loop is meant any single or looped length of suture fixed to a suture anchor at one or more points along the length of suture, as a limb of suture. In another alternate embodiment, the limb of suture consists of one or more strands of suture fixed at one end to the medial suture anchor 104 without forming a suture loop. Each suture loop 126 may be mounted to a medial suture anchor 104 by any conventional mounting means sufficient to effectively provide adequate holding strength to perform a rotator cuff repair. The suture loop 126 may be attached to the respective anchor 104 through a transverse suture passage 128 in the anchor body 112. In another alternate embodiment, each suture loop 126 may be bonded to the anchor body 112. In yet another alternate embodiment, the suture loop 7.26 is molded into the anchor body 112., The installation tool 124 may optionally include a utility suture 130 to relain each of the suture loops 126 during fixation of a medial suture anchor 104 into bone.

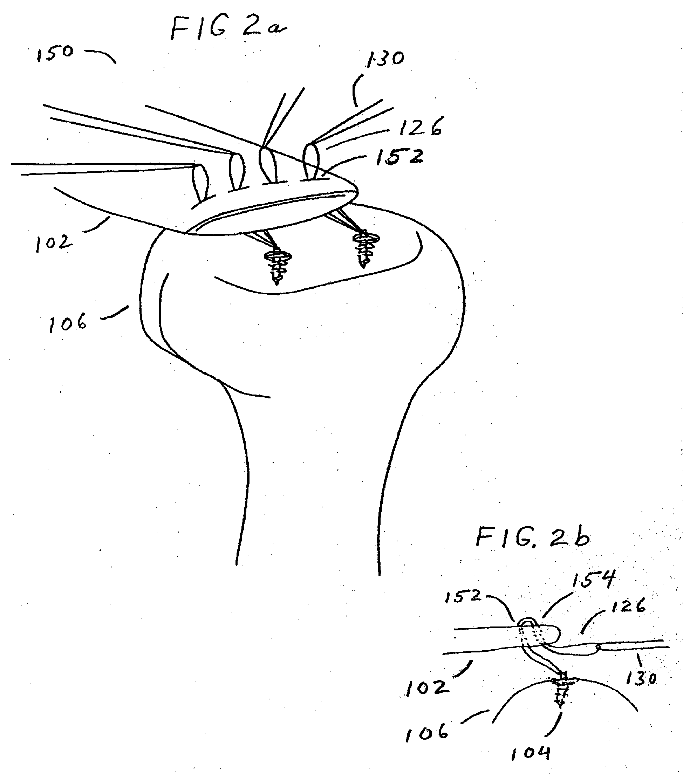

FIG. 2A illustrates surgical kit 150 of suture passing in a dual row rotator cuff repair. Each suture loop 126 is passed at location 152 through the detached rotator cuff 102 using standard arthroscopic techniques. Each suture loop 126 may be passed 152 through the rotator cuff 102 using an arthroscopic suture passer. In an alternate embodiment each suture loop 126 is passed through the rotator cuff 102 a second time at location 154, to prepare a maitress stitch 156 in the rotator cuff, as illustrated in FIG. 2B.

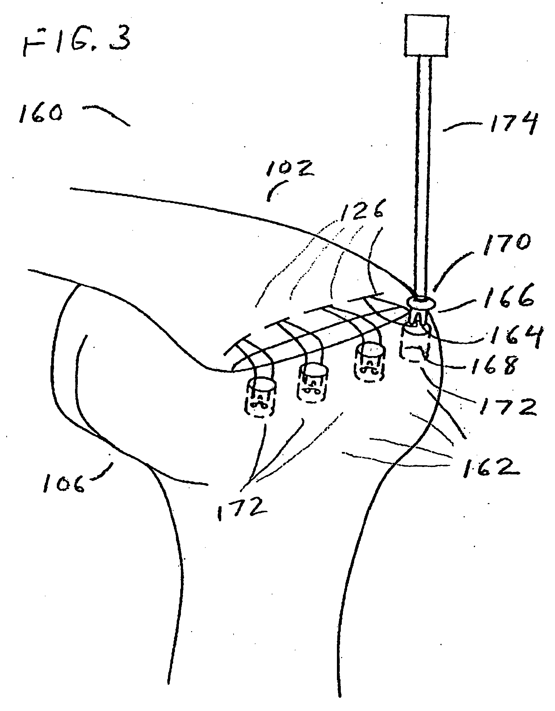

Referring to FIG. 3, a surgical kit 160 of a lateral row suture anchor installation in a dual row rotator cuff repair is shown. Each lateral suture anchor 162 includes a suture capture device 164. The suture capture device 164 provides for straightforward interconnnecting of medial and lateral anchors, without having to perform a difficult step of, for example, threading suture directly through a passage in a suture anchor after the anchor has been positioned within the patient's body at the arthroscopic surgical site, or fixed in bone. Suture capture devices can also enable the repair to be performed without tying any surgical knots. That is, the dual row repair can be a knotless repair. A variety of suture capture devices and different types of lateral anchors can be used in double row rotator cuff repair.

In one embodiment, the lateral suture anchor 162 may be an interference type fixation anchor having a body 166 with a distal end 168 and a proximal end 170, and the suture capture device 164 may be a notch positioned at the distal end 168 for snagging a respective suture loop 126. A limb of suture interconnnecting two suture anchors is also herein called a suture bridge. A bone hole 172 is formed in the humerus for receiving each lateral anchor. The proximal end 170 of the anchor is adapter for being pressed (press-fit) or pounded into bone to achieve fixation, using an elongated anchor insertion tool 174. As each lateral suture anchor 162 is pressed into the corresponding bone hole 172 for fixation into the humerus, the respective suture loop 126 is tensioned between each lateral suture anchor 162 and a corresponding medial anchor 104 (not illustrated in FIG. 3), completing the approximation of the rotator cuff 102 to the humerus 106.

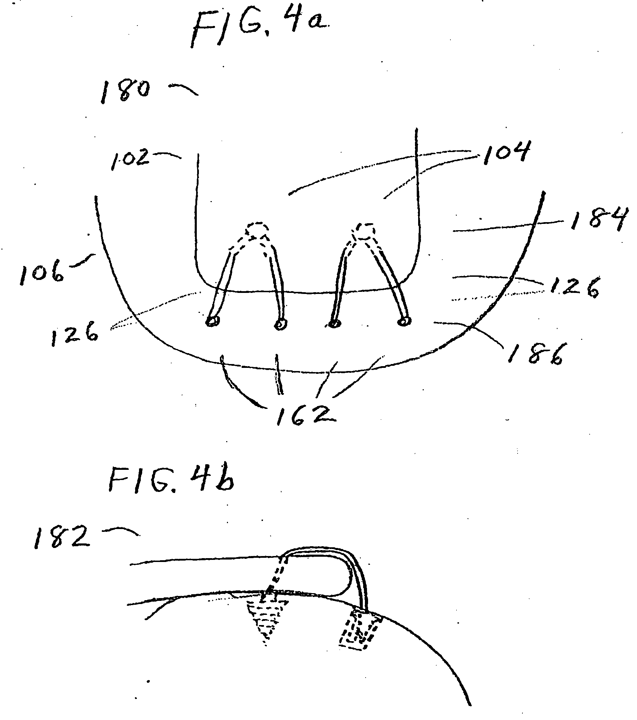

FIGS. 4A and 4B illustrate respectively, a top view of one surgical kit 180 of a completed dual row rotator cuff repair and a cross-sectional side view 182 of the completed repair. As seen in FIG. 4a, two medial anchors 104 each having two suture loops 126 attached thereto have been fixed into the humerus 106 under the rotator cuff 102, in a medial row 184 of anchors 104 and four lateral anchors 162 have been fixed into the humerus 106 in a lateral row 186 of anchors 162, positioned lateral to the medial row 184. Any number of medial and lateral anchors can be used in dual row rotator cuff repairs using the kits of the present invention, as required by the medial judgment surgeon to effect a repair. The number of medial anchors used in a dual row repair may be equal or unequal to the number of lateral anchors used in the repair. In one example, one medial anchor 104 and one lateral anchor 162 may be used to effect a dual row rotator cuff repair. In addition, any pattern of interconnection between anchors 104 and 162 in the medial row 182 and the lateral row 184, respectively, may be used. In an alternate example, all of the suture bridges may be substantially parallel to one another. Or alternately, the suture bridges may not be parallel. Optionally, two or more suture bridges cross between the medical row 182 and the lateral row 184. Two or more suture loops attached to a medial anchor may be of equal or unequal length to one another, to support symmetrical or symmetrical positioning of lateral anchors with respect to the medial anchor.

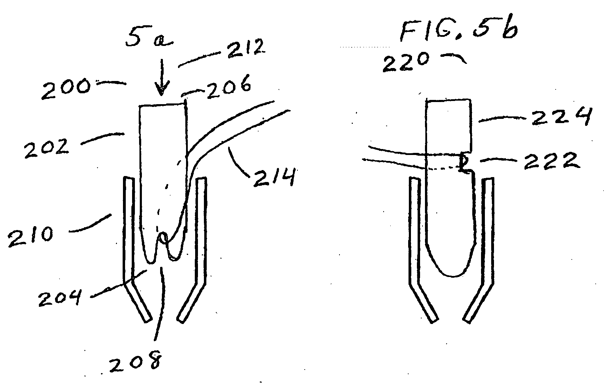

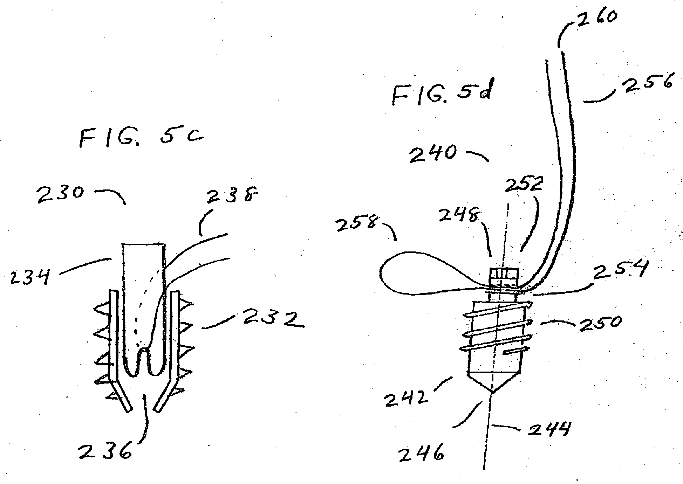

FIGS. 5A through 5D illustrate in cross -section several exemplary embodiments of suture anchors and suture capture devices associated therewith. FIG. 5A illustrates a two-part interference type fixation anchor 200. The anchor 200 includes an inner plug 202 having a distal end 204, a proximal end 206 and a suture capture notch 208 positioned at the distal end 204 of the plug 202. The anchor 200 also includes an outer sleeve 210 for insertion into bone and for receiving the plug 202. Distal motion 212 of the plug 202 into the sleeve 210 traps suture 214 that has been captured in the notch 208, between the plug 202 and the sleeve 210, and expands the sleeve 210 against bone to secure fixation of the anchor 200 in bone. The suture 214 may be locked in place by compression between two surfaces within the anchor 200. FIG. 5B illustrates an embodiment of a side-capture interference type fixation anchor 220. The side-capture interference type fixation anchor 220 resembles the anchor 200 of FIG. 5A except that a suture capture notch 222 is positioned on a side surface 224 of the side-capture anchor 220, whereas the anchor 200 illustrated in FIG 5A is distally notched. An externally threaded two-part suture anchor 230 is seen in FIG 5C, having an externally screw-threaded outer sleeve 232 for rotational engagement with bone, and an inner plug 234 including a distal suture notch 236 for capturing suture 238.

Referring to FIG. 5D, an externally threaded suture anchor 240 is seen with an associated suture capture device for dual row repairs. The anchor 240 is an externally screw-threaded type fixation anchor having a central body 242, a central longitudinal axis 244 defined through the body 242, a distal end 246, a proximal end 248 and external screw threads 250 on the body 242 about the axis 244. The screw-threaded type fixation anchor 240 also includes a proximal drive head 252 for mounting to an insertion tool, for engaging the threads 250 in bone, and a suture eyelet 254 for passing suture. In one embodiment, the eyelet 254 is a passage through the body 242 transverse to the axis 244. In another embodiment, the eyelet 254 is a loop of suture fixed to the anchor 240. Mounted to the anchor 240 is a suture threader 256 for capturing suture. The suture threader 256 includes an open, flexible suture capture loop 258 mounted through the eyelet 254, and a leader 260 for passing suture positioned within the capture loop 258. Suture positioned within the capture loop is passed through the eyelet 254 by pulling the leader 260 to withdraw the threader 256 from the eyelet 254. The suture capture loop can be made of suture, flexible wire, or another biocompatible material.

Surgical kits of the present invention provide for the surgeon performing dual row rotator cuff repairs, specific combinations of medial and lateral anchors and associated suture anchor insertion tools for fixing respective medial and lateral anchors in bone. The kits can also include tools to support the forming of bone holes for receiving one or more of a medial and a lateral suture anchor. In one embodiment, a surgical kit of the present invention includes a medial suture anchor having one or more suture loop attached whereto, a lateral suture anchor having a suture captured device associated therewith for capturing one of the one or more suture loop, a medial anchor insertion tool for fixing the medial anchor into bone, and lateral anchor insertion tool for fixing the lateral anchor in bone. The medial anchor is an externally threaded suture anchor. In a further embodiment, the kit may include a bone drill for forming a bone hole, for receiving one of the medial and the lateral anchor. In a further embodiment, the kit may include a thread-cutting bone tap for preparing the bone hole to receive engagement with external screw threads on one of the medial and the lateral anchor.

The kits of the present invention have many advantages, including but not limited to advantages associated with improved surgical outcome and facility with which a surgeon can perform arthroscopic rotator cuff repairs. One of these advantages is that arthroscopic examplary methods using the kits of the present invention are expected to better replicate the natural, biological footprint of an undamaged rotator cuff on the humerus than single row repairs, potentially improving the long-term outcome of the surgery for the patient. Two rows of suture anchors can provide a much larger area of contact for the approximation between the soft tissue and the bone. Increasing the contact area may provide enhanced opportunity for healing of the soft tissue to the bone. In addition, the larger contract area and two-dimensional distribution of anchors provided in the repair may provide increased initial fixation strength between the soft tissue and bone, thereby reducing the probability of reinjury early in the healing and rehabilitation process following surgery. Another advantage is that the two dimensional array of suture anchoring points of the dual row repairs provides purchase for a plurality of suture bridges among suture anchors, thereby further improving the approximation by pressing the tissue to the bone between the anchors, while reducing local stresses in the soft tissue to reduce pull-through or cheesewiring damage to that tissue.

Another advantage of the kits of the present invention is that knotless dual row rotator cuff repairs are provided. Arthroscopic surgeries are generally more complex and difficult for the surgeon to perform than related open surgical procedures. Arthroscopic knotless dual row repairs enhance the surgeon's effectiveness in performing the surgery, as well as beneficially reducing surgical time in the operating theater for the surgeon and the patient. The kits of the present invention allow a suture anchor having one or more attached suture loop to be directly captured by a suture capture device associated with another anchor. This suture anchor and loop design enables the surgeon to perform dual row repairs that are both knotless and do not require manual Arthroscopic threading of suture anchor cyclets. In addition, surgeries performed using kits having suture anchors having two or more attached suture loops, increases the number of interconnections along a given number of suture anchors used in the dual row repair relative to simply bridging between pairs of suture anchors, thereby potentially enhancing the security of the repair. Combinations of medial and lateral anchors, as well as anchor insertion tools and other components required to perform the dual row repairs are specific to a particular procedure. Kits of anchors, insertion tools, and other components of the present invention further support the ability of the arthroscopic surgeon to efficiently meet the patient's surgical needs for these procedures.
Many changes in details of the exemplary methods, materials, and arrangement of parts, herein described and illustrated, can be made by those skilled in the art. For example, either a medial row of anchors or a lateral row of anchors can be fixed in bone first during a dual row repair. In addition, the kits disclosed herein for dual row rotator cuff repair can provide arthroscopic repairs for any soft tissue damage requiring approximation of the damaged tissue to bone. Although the invention has been shown and described with respect to detailed embodiments thereof, it will be understood that changes may be made without departing from the scope of the invention, which is defined by the following claims.

## Claims

1. A surgical kit comprising:
a first suture anchor (104) comprising a first anchor body (112), a central longitudinal axis (114) through the body, external screw threads (120) about the axis, a distal end (116), a proximal end (118) and one or more suture loops (126) affixed proximally to the first body (112);
a second suture anchor (162; 230) having a second anchor body (166; 234) and a suture capture notch (208; 236) on the body for receiving a portion of one of the one or more suture loops;
a first anchor insertion tool (124) for fixing the first anchor into the bone; and
a second anchor insertion tool (174) for installing the second anchor into the bone,
**characterized in that**
said second suture anchor (162; 230) is an interference type fixation anchor (162), or said second suture anchor (230) has an externally screw-threaded outer sleeve (232) for rotational engagement with bone and an inner plug (234) including said suture capture notch (230) positioned distally, and
one or both of the first and second anchors (104; 162; 230) is bioabsorbable.

2. The kit of claim 1 wherein the first anchor (104) comprises two suture loops (126).

3. The kit of claim 1 or claim 2 wherein the second anchor (162) comprises a knotless anchor.

4. The kit of any preceding claim, additionally comprising at least one of a bone drill and a thread-cutting bone tap, for preparing a hole in the bone to receive one of the first anchor (104) and the second anchor (162).

5. The kit of any preceding claim, wherein the first anchor (104) and the second anchor (162) are both bioabsorbable.

## Patentansprüche

1. Chirurgisches Kit, das Folgendes umfasst:
einen ersten Nahtanker (104), der einen ersten Ankerkörper (112), eine Mittellängsachse (114) durch den Körper, Außengewinde (120) um die Achse, ein distales Ende (116), ein proximales Ende (118) und eine oder mehrere Nahtschlingen (126), die in der Nähe des ersten Körpers (112) befestigt sind, umfasst;
einen zweiten Nahtanker (162; 230), der einen zweiten Ankerkörper (166; 234) und eine Nahterfassungskerbe (208; 236) am Körper zur Aufnahme eines Abschnitts einer der einen oder mehreren Nahtschlingen aufweist;
ein erstes Ankereinführwerkzeug (124) zum Fixieren des ersten Ankers im Knochen; und
ein zweites Ankereinführwerkzeug (174) zum Einbauen des zweiten Ankers in den Knochen,
**dadurch gekennzeichnet, dass**
der zweite Nahtanker (162; 230) ein Presspassungsfixierungsanker (162) ist oder der zweite Nahtanker (230) eine mit einem Außengewinde versehene Außenhülle (232) zum Dreheingriff mit Knochen und einen inneren Stopfen (234), der die Nahterfassungskerbe (236) in distaler Position umfasst, aufweist, und
der erste oder der zweite Anker (104; 162, 230) oder beide bioabsorbierbar ist bzw. sind.

2. Kit nach Anspruch 1, wobei der erste Anker (104) zwei Nahtschlingen (126) umfasst.

3. Kit nach Anspruch 1 oder Anspruch 2, wobei der zweite Anker (162) einen knotenfreien Anker umfasst.

4. Kit nach einem vorhergehenden Anspruch, das zusätzlich einen Knochenbohrer und/oder einen gewindeschneidenden Knochengewindebohrer zum Bereitstellen eines Lochs im Knochen zur Aufnahme des ersten Ankers (104) oder des zweiten Ankers (162) umfasst.

5. Kit nach einem vorhergehenden Anspruch, wobei der erste Anker (104) und der zweite Anker (162) beide bioabsorbierbar sind.

## Revendications

1. Trousse chirurgicale comprenant :
un premier élément d'ancrage de fil de suture (104) comprenant un premier corps d'élément d'ancrage (112), un axe central longitudinal (114) traversant le corps, des filets de vis extérieurs (120) autour de l'axe, une extrémité distale (116), une extrémité proximale (118) et une ou plusieurs boucles de fil de suture (126) fixées en position proximale au premier corps (112) ;
un second élément d'ancrage de fil de suture (162 ; 230) comportant un second corps d'élément d'ancrage (166 ; 234) et une encoche de capture de fil de suture (208 ; 236) sur le corps, destinée à recevoir une partie de la ou de l'une des boucles de fil de suture ;
un premier outil d'insertion d'élément d'ancrage (124) servant à fixer le premier élément d'ancrage dans l'os ; et
un second outil d'insertion d'élément d'ancrage (174) servant à installer le second élément d'ancrage dans l'os,
**caractérisée en ce que**
ledit second élément d'ancrage de fil de suture (162 ; 230) est un élément d'ancrage (162) à fixation du type par ajustement avec serrage, ou ledit second élément d'ancrage de fil de suture (230) comporte un manchon extérieur (232) fileté extérieurement destiné à venir en prise par rotation avec un os et un bouchon intérieur (234) comprenant ladite encoche de capture de fil de suture (236) en position distale, et
le premier et/ou le second élément d'ancrage (104 ; 162, 230) sont bioabsorbables.

2. Trousse selon la revendication 1, dans laquelle le premier élément d'ancrage (104) comprend deux boucles de fil de suture (126).

3. Trousse selon la revendication 1 ou la revendication 2, dans laquelle le second élément d'ancrage (162) comprend un élément d'ancrage sans noeud.

4. Trousse selon l'une quelconque des revendications précédentes, comprenant également un foret à os et/ou un taraud à os, permettant de préparer un trou dans l'os pour la réception du premier élément d'ancrage (104) ou du second élément d'ancrage (162).

5. Trousse selon l'une quelconque des revendications précédentes, dans laquelle le premier élément d'ancrage (104) et le second élément d'ancrage (162) sont tous deux bioabsorbables.
